(19) Europäisches Patentamt / European Patent Office / Office européen des brevets

(11) **EP 2 377 515 A1**

(12) **EUROPEAN PATENT APPLICATION**

(43) Date of publication:
**19.10.2011 Bulletin 2011/42**

(51) Int Cl.:
*A61K 9/00* (2006.01)    *A61K 9/70* (2006.01)

(21) Application number: **11160476.5**

(22) Date of filing: **30.03.2011**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: 30.03.2010 JP 2010077320
           18.03.2011 JP 2011061515

(71) Applicant: **Nitto Denko Corporation
Osaka 567-8680 (JP)**

(72) Inventors:
 • **Sakamato, Sachiko
  Osaka 567-8680 (JP)**

 • **Okazaki, Arimichi
  Osaka 567-8680 (JP)**
 • **Akemi, Hitoshi
  Osaka 567-8680 (JP)**
 • **Iwao, Yoshihiro
  Osaka 567-8680 (JP)**
 • **Matsuoka, Kensuke
  Osaka 567-8680 (JP)**
 • **Hanatani, Akinori
  Osaka 567-8680 (JP)**

(74) Representative: **Duckworth, Timothy John
  J.A. Kemp & Co.
  14 South Square
  Gray's Inn
  London WC1R 5JJ (GB)**

(54) **Patch preparation and production method thereof**

(57) Provided is a patch preparation containing: a support; and an adhesive layer containing a drug on the surface of the support, wherein a crystal of the drug is formed in the adhesive layer immediately after application of a physical stimulation to the adhesive layer, while a crystal of the drug is formed during preservation after the physical stimulation.

According to the present invention, a patch preparation which does not require increase in the area and thickness of an adhesive layer, achieves sufficiently high skin permeation amount of a drug, shows good adhesiveness to the skin and permits a long-term adhesion, and a production method thereof can be provided.

EP 2 377 515 A1

**Description**

TECHNICAL FIELD OF THE INVENTION

The present invention relates to a patch preparation comprising an adhesive layer containing a drug on one surface of a support, and a production method thereof.

BACKGROUND OF THE INVENTION

To increase the skin permeation amount of a drug during application of a patch preparation to the skin, the amount of a drug in the patch preparation may be increased. To increase the amount of a drug in an adhesive layer, the area and thickness of the adhesive layer of a patch preparation may be increased. However, such embodiment deteriorates the handling property of the patch preparation. Since transfer of a drug into the skin is known to depend on the concentration of the drug in an adhesive layer of a patch preparation, the skin permeation amount of a drug may be increased by increasing the drug concentration of the adhesive layer.

On the contrary, a patch preparation with a higher drug concentration tends to readily form a crystal of the drug. When the amount of the crystal of the drug is small, it does not adversely influence the usefulness of a patch preparation. However, even when the amount of the crystal formation of the drug is small, inconveniences may be induced such as patients' concern about the quality degradation, thereby making them hesitate to use the preparation and the like. When the amount of the crystal formation of the drug is high, not only the drug release characteristic changes but also an adhesive area decreases when the crystal covers the surface of the adhesive layer. As a result, the adhesiveness to the skin may decrease and the patch preparation may not be able to maintain the adhesiveness for a long time.

Therefore, a patch preparation capable of increasing a drug skin permeation amount, reducing the area and thickness of an adhesive layer of the patch preparation, thereby improving handling property and compliance by decreasing irritation and uncomfortableness during adhesion and the like, and maintaining the adhesiveness to the skin for a long time is desired.

JP-B-3566301, National Publication of International Patent Application No. 2006-513195, JP-B-2610314, JP-B-4166276, and National Publication of International Patent Application No. 2001-514213 teach that crystal formation of a drug can be suppressed by heating a laminated sheet containing a drug-containing adhesive layer, or a patch preparation precursor, or a laminated sheet and a patch preparation precursor. In all these publications, however, since the heating temperature is not less than the melting point of a drug, the drug is melted as well as exposed to a high temperature, thus possibly resulting in denaturation of the drug. Moreover, these publications are silent on the problems of crystal formation of drug that may occur when a patch preparation is actually applied to the skin, even when a crystal of the drug was not formed during the production steps of the patch preparation.

SUMMARY OF THE INVENTION

It is therefore an object of the present invention to provide a patch preparation which does not require increase in the area and thickness of an adhesive layer, achieves sufficiently high skin permeation amount of a drug (i.e., having superior drug release characteristic), shows good adhesiveness to the skin and permits a long-term adhesion, and a production method thereof.

In addition, the present invention aims to provide a patch preparation which does not give rise to patients' concern about the quality degradation during use, achieves sufficiently high skin permeation amount of a drug (i.e., having superior drug release characteristic), shows good adhesiveness to the skin and permits a long-term adhesion, and a production method thereof.

To achieve the above-mentioned object, the present invention has the following constitution.
Accordingly, the present invention relates to

[1] a patch preparation comprising: a support; and an adhesive layer containing a drug on one surface of the support, wherein a crystal of the drug is not formed in the adhesive layer immediately after application of a physical stimulation to the adhesive layer, while a crystal of the drug is formed during preservation after the physical stimulation.
[2] the patch preparation of [1], wherein the drug in the adhesive layer is dissolved in the adhesive layer,
[3] the patch preparation of [1] or [2], wherein a crystal of the drug is formed not less than 24 hr and within 6 months when the patch preparation is preserved at not more than 25˚C after application of physical stimulation,
[4] the patch preparation of [1] or [2], wherein a crystal of the drug is formed not less than 24 hr and within 6 months when the patch preparation is preserved in an environment of 25˚C±5˚C, relative humidity 60%RH±5%RH after application of physical stimulation, and
[5] a method of producing the patch preparation of any one of the above-mentioned [1] - [4], comprising a step of heating the adhesive layer containing the drug to a temperature less than the melting point of the drug.

In addition, the present invention relates to

[6] a method of producing a patch preparation comprising: a support; and an adhesive layer containing a drug on the surface of the support, which is provided on one surface of a support, comprising
a step of cutting a laminated sheet wherein support/adhesive layer containing a drug/release liner are laminated in this order into the form and size of a patch preparation to give a patch preparation precursor, and a step of heating the patch preparation precursor at not less than 40°C and less than 100°C and less than the melting point of the drug,
[7] the method of the above-mentioned [6], wherein the patch preparation precursor is heated for 3 - 120 hr, and
[8] the method of the above-mentioned [6] or [7], wherein the concentration of the drug in the adhesive layer is 100 - 300% to the saturating concentration of the drug in the adhesive layer.

According to the present invention, a patch preparation which does not require increased area and thickness of an adhesive layer, achieves sufficiently high skin permeation amount of a drug, shows good adhesiveness to the skin and permits a long-term adhesion, and a production method thereof can be provided.
In addition, according to the present invention, a patch preparation which does not give rise to patients' concern about the quality degradation during use, achieves sufficiently high skin permeation amount of a drug (i.e., having superior drug release characteristic), shows good adhesiveness to the skin and permits a long-term adhesion can be obtained.

DETAILED DESCRIPTION OF THE INVENTION

The patch preparation of the present invention comprises an adhesive layer containing a drug, which is formed on one surface of a support, and is characterized in that a crystal of the drug is not formed in the adhesive layer immediately after application of a physical stimulation to the adhesive layer of the patch preparation; while a crystal of the drug is formed during preservation after the physical stimulation.
When the crystal of the drug is formed in the adhesive layer from immediately after physical stimulation of the adhesive layer, even if the amount of the crystal of the drug is small and the crystal does not adversely influence the usefulness of the patch preparation, patients may have concern about the quality degradation and hesitate to use the preparation. When the amount thereof is high, not only the drug release characteristic changes but also the crystal covers the surface of the adhesive layer and reduces the adhesive area. As a result, the adhesiveness to the skin may decrease and the patch preparation may not be able to maintain the adhesiveness for a long time.
In the present invention, "a crystal of the drug is not formed in the adhesive layer immediately after application of a physical stimulation to the adhesive layer" means a crystal of the drug is not formed in the adhesive layer during preservation for not less than 0 hr and less than 24 hr from the application of physical stimulation on the adhesive layer.
Here, "a crystal of the drug is not formed in the adhesive layer" basically means that a crystal of the drug is not found in the adhesive layer by visual observation, and in a preferable embodiment, it means that a crystal of the drug is not found in the adhesive layer both by visual observation and microscopic observation (maximum magnification x50.0). In addition, "physical stimulation" means that the adhesive layer of a patch preparation is pierced through with a toughened and sharp-pointed part of a tool, for example, the adhesive layer of a patch preparation is pierced through with the edge of a cutter, the adhesive layer is cut with the toughened and sharp-pointed part and the like.
In the patch preparation of the present invention, a crystal of the drug is preferably formed during the preservation term of not less than 24 hr (=1 day) and within 6 months, more preferably not less than 1 week (=7 days) and within 6 months, from the application of physical stimulation on the adhesive layer.
When a crystal of the drug is formed less than 24 hr from the application of physical stimulation on the adhesive layer (that is, a crystal of the drug is formed immediately after application of physical stimulation on the adhesive layer), the concentration of the drug in the adhesive layer is too high. In this case, crystal formation of the drug might have occurred when actually using the patch preparation, possibly causing difficulty in use. As a result, patients may have concern about the quality degradation during use and, when a crystal precipitates on the surface of the adhesive layer, the area having adhesiveness decreases and the adhesiveness to the skin may decrease.
On the other hand, when crystal formation of the drug requires longer than 6 months from the application of physical stimulation, the concentration of the drug in the adhesive layer is assumed to be low. In this case, a sufficient skin permeation amount of the drug may be difficult to achieve.
In the patch preparation of the present invention, a crystal of the drug is not formed in the adhesive layer immediately after application of a physical stimulation to the adhesive layer, while a crystal of the drug is formed during preservation after the physical stimulation. The reason therefor is assumed that the adhesive layer containing the drug at a reasonably high concentration forms a crystal core of the drug when the adhesive layer is subject to a physical stimulation; whereby the core of the crystal of the drug gradually grows during preservation of the patch preparation.

The "preservation" in the present specification means that a patch preparation is stood under an environment of temperature 25°C±5°C and relative humidity 60%RH±5%RH.

The patch preparation of the present invention is preferably free of a crystal of a drug in an adhesive layer immediately after production. When even a slight amount of a crystal of a drug is contained in the adhesive layer, the crystal may grow during preservation even in the absence of a particular physical stimulation on the patch preparation and produce a large amount of the crystal of the drug. Therefore, the drug is preferably dissolved in the adhesive layer immediately after production.

In the patch preparation of the present invention, the drug is not particularly limited, and a transdermally absorbable drug, which can be administered to mammals such as human and the like through the skin, is preferable. Specific examples of such drug include general anesthetics, hypnotic sedatives, antiepileptic drugs, antipyretic analgesic antiphlogistic drugs, anti-vertiginous drugs, psychoneurotic drugs, central neurological drug, topical anesthetics, skeletal muscle relaxants, autonomic drugs, antispasmodic drugs, anti-parkinsonian drugs, anti-histamine drugs, cardiac stimulants, drugs for arrhythmia, diuretic, hypotensive drug, vasoconstrictor, coronary vasodilator, peripheral vasodilators, arteriosclerosis drugs, drugs for circulatory organ, anapnoics, antitussive expectorant, hormone drugs, external drugs for purulent diseases, analgesic-antipruritic-styptic-antiinflammatory drugs, drugs for parasitic skin diseases, hemostatic drugs, gout treatment drugs, drugs for diabetes, anti-malignant tumor agents, antibiotic, chemical therapy agents, narcotic, quit smoking aids and the like.

To sufficiently afford the effect of the present invention, the drug is advantageously a solid drug at room temperature (25°C). The solid drug at room temperature means a drug having its melting point higher than 25°C as measured by DSC (differential scanning calorimeter). The melting point here is an extrapolation melting start temperature in a DSC curve measured according to JIS K 7121-1987 and using DSC. The extrapolation melting start temperature is a temperature at the intersection point between a straight line extending from the baseline on the low temperature side toward the high temperature side, and a tangent line to the curve on the low temperature side of the meltpeak at the point of maximum gradient.

The drug of which crystallization peak in the measurement by DSC is not detected is preferable. Using such a drug, formation of the drug crystal in the adhesive layer immediately after production of a patch preparation (before preservation of the patch preparation) can be certainly suppressed. Since a drug of which crystallization peak is not detected does not easily form its crystal structure, such a drug is assumed to be less apt to form a crystal in the adhesive layer.

The crystallization peak means an exothermic peak due to crystallization of a drug as measured according to JIS K 7121-1987 and using DSC. To be specific, 5 mg - 10 mg of a drug is measured in a measurement container, and the peak is measured by the following temperature program to give a DSC curve. The drug is maintained at a temperature lower by 50°C than the melting point of the drug for 10 min, heated at a temperature raise rate of 2°C per minute, and maintained at a temperature higher by 30°C than the melting point for 3 min. Thereafter, the temperature is decreased at a rate of 2°C per minute, and the drug is maintained at a temperature lower by 30°C than the glass transition temperature of the drug for 10 min, and then heated again at a rate of 2°C per minute to a temperature higher by 30°C than the melting point of the drug.

The glass transition temperature is a middle point of step-like changes accompanying the glass transition of the drug in the above-mentioned DSC curve. The middle point of the step-like changes is a temperature at an intersection point between a straight line located at an equal distance from two straight lines, which are extended baselines before and after the step-like changes, and the curve of the step-like changing part of glass transition.

The aforementioned melting point is an extrapolation melting start temperature of an endothermic peak accompanying drug melting in the above-mentioned DSC curve.

Examples of the drug of which crystallization peak by DSC analysis is not detected include donepezil, miconazole, diltiazem, scopolamine, ketoprofen, indomethacin, capsaicin, ibuprofen, isosorbide dinitrate, diclofenac, rotigotine and the like.

In one embodiment of the present invention, the drug is donepezil and/or a pharmaceutically acceptable salt thereof. In another embodiment of the present invention, the drug is a drug other than donepezil and/or a pharmaceutically acceptable salt thereof.

The drug is present in the adhesive layer in an amount sufficient to provide desired results in the treatment of disease, condition or disorder, for example, desired therapeutic effect, (to be referred to as an effective amount in the present specification). The effective amount of the drug means, for example, an amount of the drug that provides a concentration of the drug in blood lower than a toxic level and sufficient to provide a selected effect over a predetermined time.

Such amount can be easily determined by those of ordinary skill in the art.

The weight concentration of the drug in the adhesive layer is not particularly limited as long as it is within the range of the above-mentioned effective amount, can ensure sufficient skin permeation amount of the drug, and does not impair adhesion property of the adhesive. For example, the weight concentration is 0.1 - 60 wt%, preferably 0.5 - 40 wt%, relative to the whole adhesive layer. When the weight concentration is less than 0.1 wt%, the treatment effect may be insufficient, and when it exceeds 60 wt%, the content of an adhesive constituting the adhesive layer becomes small, and sufficient skin adhesiveness may not be achieved, which is economically disadvantageous.

The weight concentration (wt%) of the drug in an adhesive layer is a ratio of the weight concentration (wt%) to the saturating concentration (wt%) of the drug in the adhesive layer, and is preferably 100 - 300%, more preferably 100 - 250%. When the ratio of the weight concentration to the saturating concentration [(weight concentration/saturating concentration)$\times$100(%)] is less than 100%, sufficiently high skin permeation amount of the drug tends to be difficult to achieve, since the drug concentration of the adhesive layer is low. When it exceeds 300%, crystal formation of the drug tends to occur even if a physical stimulation is not applied to the adhesive layer during the production step of the patch preparation or after production thereof.

In addition, a saturating concentration of a drug in an adhesive layer can be determined by the following operation. Patch preparations with an adhesive layer having an incrementing drug concentration by 1 wt% are prepared, a liner on the adhesive layer of the respective patch preparations is detached, a small amount of a drug is left standing on the exposure face of the respective adhesive layers, and the liner is laminated again on the adhesive layer. The thus-produced patch preparations are stored in an incubator at 25˚C for 6 months, and the size change of the drug left on the adhesive layer is observed every month under an optical microscope (magnification: 500-fold). When the drug size increases, the drug concentration is judged to have exceeded the saturating concentration, when the drug size decreases, the drug concentration is judged to be less than the saturating concentration, and when the drug size does not change, the drug concentration is judged to be near the saturating concentration.

This measurement method is based on the following facts. When the drug concentration is drastically smaller than the saturating concentration, the drug stood dissolves in the adhesive layer to reduce the drug size, when the drug concentration is higher than the saturating concentration, crystal grows from the drug stood to increase the drug size, and when the drug concentration is near the saturating concentration, the drug size does not change since dissolution and growth of the drug stood are equilibrated.

For example, when the size of the drug stood becomes small in a patch preparation having a drug concentration of 1 - 5 wt%, does not change in a patch preparation having a drug concentration of 6 - 7 wt%, and increases in a patch preparation having a drug concentration of 8 - 10 wt%, the saturating concentration of the drug in an adhesive layer is 7% (wt%). When the saturating concentration is judged to be less than 1% according to this method (when the size of the drug stood increases in a patch preparation having a drug concentration of 1 wt%), a patch preparation having an incrementing drug concentration by 0.2 wt% within the range of 0 - 1 wt% is prepared, and evaluated in the same manner as above, and a saturating concentration is determined.

In the patch preparation of the present invention, the support is not particularly limited, and various plastic films, non-woven fabric, paper, woven fabric, knitted fabric, metal foil, and laminate of these can be used. When desired, a metal may be applied thereon by vapor deposition. The plastic film is not particularly limited, and various films formed from a polyvinyl chloride single substance, or a copolymer of a monomer such as ethylene, propylene, vinyl acetate, acrylic acid, acrylic acid ester, methacrylic acid, methacrylic acid ester, acrylonitrile, styrene, vinylidene chloride and the like, and other monomer, or an olefin system polymer such as polyethylene, polypropylene, ethylene-vinyl acetate copolymer and the like; a polyester system polymer such as polyethylene terephthalate, polyetherpolyester and the like; a polyamide system polymer such as polyetherpolyamide block polymer and the like, and the like can be used. The thickness of the support is generally 10 - 500 $\mu$m, preferably 10 - 200 $\mu$m.

The adhesive layer is a layer-like structure containing a polymer and shows adhesiveness at ambient temperature (25˚C). In view of the handling property of the preparation, the thickness of the adhesive layer is preferably 10 - 500 $\mu$m, more preferably 15 - 300 $\mu$m, particularly preferably 20 - 250 $\mu$m.

The polymer to be contained in an adhesive layer is not particularly limited, and examples thereof include acrylic polymers comprising (meth)acrylic acid ester system polymer; rubber polymers such as styrene-diene-styrene block copolymer (e.g., styrene-isoprene-styrene block copolymer, styrenebutadiene-styrene block copolymer), polyisoprene, polyisobutylene, polybutadiene and the like; silicone polymers such as silicone rubber, dimethylsiloxane base, diphenylsiloxane base and the like; vinyl ether polymers such as polyvinyl methylether, polyvinyl ethylether, polyvinyl isobutylether and the like; vinyl ester polymers such as vinyl acetate-ethylene copolymer and the like; polyester polymers comprising an carboxylic acid component such as dimethylterephthalate, dimethylisophthalate, dimethylphthalate and the like and a polyvalent alcohol component such as ethylene glycol and the like, and the like.

As an acrylic polymer, one containing alkyl (meth)acrylate as a main component, and obtained by copolymerization with a functional monomer, is preferable. That is, a copolymer containing 50 - 99 wt% (preferably 60 - 95 wt%) of a monomer component comprised of alkyl (meth)acrylate, and the remaining monomer component comprised of a functional monomer is preferable. The main component here means a monomer component contained in a proportion of not less than 50 wt% of the total weight of the monomer component constituting the copolymer.

In alkyl (meth)acrylate (hereinafter to be also referred to as the main component monomer), the alkyl group generally consists of a straight chain of branched alkyl group having a carbon number of 4 - 13 (e.g., butyl, pentyl, hexyl, heptyl, octyl, 2-ethylhexyl, nonyl, decyl, undecyl, dodecyl, tridecyl and the like), and one or more kinds thereof are used.

A functional monomer has, in a molecule, at least one unsaturated double bond relating to a copolymerization reaction and a functional group on the side chain. Examples thereof include carboxyl group-containing monomers such as (meth)acrylic acid, itaconic acid, maleic acid, maleic anhydride and the like, hydroxyl group-containing monomers such as hydroxyethyl (meth)acrylate, hydroxypropyl (meth)acrylate and the like; sulfo group-containing monomers such as styrene sulfonic acid, ally sulfonic acid, sulfopropyl(meth)acrylate, (meth)acryloyloxynaphthalene sulfonic acid, acrylamido methyl propane sulfonic acid and the like; amino group-containing monomers such as (meth)acrylic acid aminoethyl ester, (meth)acrylic acid dimethylaminoethyl ester, (meth)acrylic acid tert-butylaminoethyl ester and the like; amide group-containing monomers such as (meth)acrylamide, dimethyl(meth)acrylamide, N-methylol(meth)acrylamide, N-methylolpropane(meth)acrylamide, N-vinylacetamide and the like; and alkoxyl group-containing monomers such as (meth)acrylic acid methoxyethyl ester, (meth)acrylic acid ethoxyethyl ester, (meth)acrylic acid methoxyethyleneglycol ester, (meth)acrylic acid methoxydiethyleneglycol ester, (meth)acrylic acid methoxypolyethylene glycol ester, (meth)acrylic acid methoxypolypropyleneglycol ester, (meth)acrylic acidtetrahydrofurylester and the like.

One or more kinds of the functional monomers can be used and, among them, a carboxyl group-containing monomer is preferable, and (meth)acrylic acid is particularly preferable, from the aspects of pressure-sensitive adhesiveness of the adhesive layer, cohesiveness, the release characteristic of the drug contained in the adhesive layer, and the like.

As an acrylic polymer, a copolymer of the above-mentioned alkyl (meth)acrylate (main monomer component) and a functional monomer, which is further copolymerized with other monomer, can also be used.

Examples of other monomer include (meth)acrylonitrile, vinyl acetate, vinyl propionate, N-vinyl-2-pyrrolidone, methylvinylpyrrolidone, vinylpyridine, vinylpiperidone, vinylpyrimidine, vinylpiperazine, vinylpyrrole, vinylimidazole, vinylcaprolactam, vinyloxazole and the like, and one or more kinds thereof can be used.

The amount of other monomer to be used is generally preferably about 0 - 40 wt%, more preferably about 10 - 30 wt%, relative to the total weight of alkyl (meth)acrylate (main monomer component) and a functional monomer.

As a specific preferable example of the acrylic polymer, a terpolymer of 2-ethylhexyl acrylate as alkyl (meth)acrylate, acrylic acid and N-vinyl-2-pyrrolidone is preferable, and a copolymer obtained by copolymerization of 2-ethylhexylacrylate, acrylic acid and N-vinyl-2-pyrrolidone at a weight ratio of 40 - 99.9:0.1 - 10:0 - 50 is more preferable, since adhesiveness to the human skin is fine, and adhesion and detachment can be repeated easily.

Of the rubber polymers, a rubber polymer containing at least one kind selected from polyisobutylene, polyisoprene and styrene-dienestyrene block copolymer (styrene-butadienestyrene block copolymer (SBS), styrene-isoprene-styrene block copolymer (SIS) etc.) as a main component is preferable. Since drug stability is high, and the necessary adhesive force and cohesion strength can be simultaneously achieved, a mixture of a rubber polymer containing a high molecular weight polyisobutylene with a viscosity average molecular weight of 500,000 - 2,100,000, and a low molecular weight polyisobutylene with a viscosity average molecular weight of 10,000 - 200,000 at a weight ratio of 95:5 - 5:95 is particularly preferable.

When a rubber polymer is used, a tackifier is further preferably added to impart adhesiveness at ambient temperature to an adhesive layer. As a tackifier, those known in the relevant technical field can be appropriately selected for use. Examples of the tackifier include petroleum resin (e.g., aromatic petroleum resin, aliphatic petroleum resin), terpene resin, rosin resin, coumarone indene resin, styrene resin (e.g., styrene resin, poly(α-methylstyrene) etc.), hydrogenated petroleum resin (e.g., alicyclic saturated hydrocarbon resin etc.) and the like. Among these, an alicyclic saturated hydrocarbon resin is preferable since it improves stability of the drug. Tackifier may be a combination of one or more kinds thereof. The amount of the tackifier is generally 33 - 300 wt%, preferably 50 - 200 wt%, relative to the total weight of the rubber polymer.

When desired, for example, for adjustment of adhesiveness, acceleration of transdermal absorption of a drug and the like, the adhesive layer may contain an organic liquid component. The organic liquid component is an organic compound which is liquid at room temperature (25°C) and plasticizes an adhesive layer. Examples thereof include glycols such as ethylene glycol, diethylene glycol, triethylene glycol, propylene glycol, polyethylene glycol, polypropylene glycol and the like; fats and oils such as olive oil, castor oil, squalene, lanolin and the like; organic solvent such as ethyl acetate, ethyl alcohol, dimethyldecyl sulfoxide, methyloctyl sulfoxide, dimethyl sulfoxide, dimethylformamide, dimethylacetamide, dodecylpyrrolidone, isosorbitol and the like; liquid surfactant; plasticizers such as octyldodecanol, diisopropyladipate, phthalic acid ester, diethylsebacate, triethyl citrate, acetylcitric acid tributyl and the like; hydrocarbons such as liquid paraffin and the like; ethoxylated stearyl alcohol; fatty acid ester; glycerol acid ester and the like.

Of these, fatty acid ester, glycerol acid ester (mono, di, or triglyceride), octyldodecanol, acetylcitric acid tributyl, liquid paraffin and the like are preferable.

To maintain compatibility with the polymer in an adhesive layer and prevent volatilization in the heating step during preparation of a patch preparation, a fatty acid ester comprised of a higher fatty acid having a carbon number of 12 - 16, (more preferably 12 - 14) and a lower monovalent alcohol having a carbon number of preferably 1 - 4 is preferably used. Examples of the higher fatty acid having a carbon number of 12 - 16 include lauric acid, myristic acid, palmitic acid and the like, and examples of the lower monovalent alcohol having a carbon number of 1 - 4 include methyl alcohol, ethyl alcohol, propyl alcohol, isopropyl alcohol and the like.

As the glycerol acid ester (mono, di, or triglyceride), glycerol middle chain (carbon number 8 - 12) fatty acid ester is preferable, and may be any one of monoglyceride, diglyceride and triglyceride, or a mixture of two or more kinds thereof, with preference given to triglyceride. As the medium-chain triglyceride, a triglyceride wherein only one kind of middle chain fatty acid is ester bonded to glycerol (e.g., caprylic acid triglyceride wherein only caprylic acid is ester bonded to glycerol, capric acid triglyceride wherein only capric acid is ester bonded and the like) may be used, or a triglyceride wherein multiple kinds of middle chain fatty acid are ester bonded to glycerol (e.g., (caprylic acid-capric acid) triglyceride wherein caprylic acid and capric acid are ester bonded to glycerol, (caprylic acid-capric acid-lauric acid) triglyceride wherein caprylic acid, capric acid and lauric acid are ester bonded to glycerol, and the like) may be used.

When desired, the adhesive layer may be applied to physical crosslinking by irradiation such as ultraviolet irradiation, electron beam irradiation and the like, or chemical crosslinking treatment using an isocyanate compound such as trifunctional isocyanate and the like and various crosslinking agents such as organic peroxide, organic metal salt, metal alcoholate, metal chelate compound, multifunctional compound (multifunctional external crosslinking agent, or multifunctional internal crosslinkable monomer such as diacrylate, dimethacrylate and the like) and the like to give a crosslinked adhesive layer. The crosslinked adhesive layer containing an organic liquid component is preferable since it has appropriate skin adhesiveness due to its gel state, and cohesive property that does not leave an adhesive residue on detachment.

To protect an adhesive face of the adhesive layer before use, a release liner may be applied. While the release liner is not particularly limited, plastic films such as a polyester film, specifically a polyethylene terephthalate film and the like, and a laminate film thereof can be mentioned. Since the number of kinds is many, has an appropriate thickness as a patch preparation, and materials are easy to select, a polyester film, particularly a polyethylene terephthalate film is preferable.

In consideration of easy processing and processing precision, a release liner having a uniform thickness is preferable. While the thickness is not particularly limited, from the aspects of easy production of a patch preparation, cost of release liner, portability of patch preparation, operability and the like, it is preferably 25 $\mu$m - 200 $\mu$m, more preferably 50 $\mu$m - 150 $\mu$m. To further facilitate detachment of the release liner from the adhesive layer, a surface release treatment (surface treatment with silicone release agent, fluorine release agent, wax and the like) may be applied to the surface on the adhesive layer side of the release liner.

When desired, the patch preparation of the present invention can be packed in a packaging container known per se. As the packaging container, one made of a resin film, a metal foil or a laminate film thereof is generally used.

While a method of producing the patch preparation of the present invention, namely, a patch preparation wherein a crystal of a drug is not formed in the adhesive layer immediately after application of a physical stimulation to the adhesive layer, but formed during preservation after the physical stimulation, is not particularly limited, the following method is preferable.

A release liner is prepared, an adhesive layer is laminated on one surface of the release liner, and a support is laminated on the adhesive layer to give a laminated sheet. Alternatively, a support is prepared, an adhesive layer is laminated on one surface of the support, and a release liner is laminated on the adhesive layer to give a laminated sheet. That is, a laminated sheet wherein support/adhesive layer containing a drug/release liner are laminated in this order is obtained. A method of lamination is not particularly limited, and coating, adhesion, heat-sealing, fusion splicing and the like can be mentioned. The method of laminating an adhesive layer on a release liner or a support preferably includes steps of preparing a composition for forming an adhesive layer, which contains a drug, a polymer, an organic solvent and the like, applying same on a release liner or a support, and removing the organic solvent by drying.

A laminated sheet containing the obtained adhesive layer is cut into the form of a patch preparation in a cutting step and, where necessary, packed in a packing container to give a patch preparation precursor. The patch preparation precursor here means a product which has been processed from the laminated sheet into the form and size of a patch preparation by cutting processing and, where necessary, packed in a packing container, and prior to a heating step. In this case, the obtained laminated sheet as a whole can be subjected to the cutting step to cut the adhesive layer. The cutting step can be performed by cutting with an edge tool such as a cutter, punching edge and the like.

The obtained patch preparation precursor is heated to give a patch preparation. In the above-mentioned cutting step, an impact (physical stimulation) is applied to the adhesive layer, which is assumed to cause formation of a crystal core of the drug in the adhesive layer. By heating the adhesive layer to a particular temperature, preferably not higher than the melting point of the drug, the crystal core can be dissolved. Preferable heating temperature and time are those sufficient to completely dissolve all crystal cores of the drug in the adhesive layer. When the crystal core is once completely dissolved in the adhesive layer, crystal is not easily formed and the crystal formation can be stably suppressed.

The patch preparation precursor is preferably heated immediately after obtaining the patch preparation precursor. When the patch preparation precursor is left standing for a long time before heating, crystal cores of the drug generated in the adhesive layer due to an impact (physical stimulation) in the cutting step to give the patch preparation precursor may grow too large while being left standing to be completely dissolved in the heating step. While an acceptable time period from obtaining a patch preparation precursor to heating thereof cannot be determined easily since it depends on the drug concentration of the adhesive layer, it is the period when the grown crystal cannot be visually confirmed.

The heating temperature needs to be not less than 40˚C and less than 100˚C and less than the melting point of the drug, which is preferably not less than 40˚C and less than 91˚C and less than the melting point of the drug, more preferably not less than 54˚C and less than 84˚C and less than the melting point of the drug. When the heating temperature is not less than the melting point of the drug, the drug may be degraded, and the packing container may be melted. On the other hand, when the heating temperature is much lower than the melting point, a temperature for the saturating concentration may not be reached, leaving the drug in crystals in the adhesive layer. In addition, even if the heating temperature is less than the melting point of the drug, when the heating temperature exceeds 100˚C, inconveniences such as possible denaturation of an adhesive component in the adhesive layer, melting of a packing container when it is used and the like may occur.

While the heating time is not particularly limited as long as it is sufficient to dissolve a drug in the adhesive layer, it is, for example, 3 - 120 hr, preferably 6 - 72 hr, more preferably 9 - 72 hr. When the heating time is too long, a decrease in the drug content due to degradation of the drug, and color change of the adhesive layer caused by impurities resulting from degradation of the drug, such as oxide, decomposition product and the like, are feared. On the other hand, when it is too short, crystal cores cannot be dissolved, and crystal formation cannot be prevented.

A cooling step may be added after a heating step. Since additional facility and step are not required, cooling is preferably natural cooling at environmental temperature.

The thus-produced patch preparation is stored until shipping. During the storage, a patch preparation is generally left in an environment of temperature 5 - 25˚C, relative humidity 10 - 75%RH. When the drug easily forms a hydrate, the patch preparation is preferably stored under dry conditions (relative humidity: 10%RH±5%RH). To avoid doubt, it is noted that "under dry conditions" refers to the conditions of atmosphere to be in direct contact with the patch preparation, and does not refer to the atmosphere with which the patch preparation does not directly contact, for example, the atmosphere outside the package body and when the patch preparation is housed in a package body.

The drug that easily forms a hydrate is a drug having properties to form a hydrate crystal at room temperature (25˚C), relative humidity 0 - 98%RH. A number of drugs that easily form a hydrate are already known, and specific examples thereof include scopolamine, citric acid, theophylline and pharmaceutically acceptable salts thereof and the like. Whether a drug easily forms a hydrate can be known by the following test method.

[Test method 1]

According to solid-water interaction: absorption and desorption isothermal lines and measurement of water activity described in draft revision (as of December 10, 2010) scheduled for listing in Supplement 1 to the Japanese Pharmacopoeia, 16th Edition, an adsorption isothermal line and a desorption isothermal line of the drug are formed using a water vapor automatic adsorption desorption apparatus. In the measurement, relative humidity is increased from 0 to 98%RH under atmospheric pressure at 25˚C at 5 - 10% distance, then decreased from 98 to 0%RH at 5 - 10% distance in the same manner, and balancing weight is measured in each atmosphere. With regard to the obtained adsorption isothermal line and desorption isothermal line, the following are confirmed:

(i) in the adsorption isothermal line, the maximum change in the weight of the drug before measurement is not less than 1.0 wt%,
(ii) at least in the adsorption isothermal line, an inflection point is observed, and
(iii) hysteresis is observed in the adsorption isothermal line and the desorption isothermal line.

The "hysteresis is observed" means that the difference between the inflection point of the adsorption isothermal line and that of the desorption isothermal line is not less than 10%.

[Test method 2]

TG curve of a drug is formed according to JIS K 7120-1987, and the weight decrease rate is calculated. The weight decrease rate means change in the weight of the drug at the completion of the measurement to the weight of the drug before measurement, and whether the weight decrease rate is not less than 1.0 wt% is confirmed.

[Test method 3]

DSC curve of a drug is formed according to the Japanese Pharmacopoeia, 15th Edition, General Test, 2.52 Thermal Analysis, Method 1. The temperature rise rate during the measurement is the same as in Test method 2. Whether an endothermic peak (downward convex peak) is observed in the DSC curve at the temperature of the inflection point in the TG curve formed in Test method 2 is confirmed.

A drug that satisfies all of (i) - (iii) in the above-mentioned Test method 1, Test method 2 and Test method 3 is judged as a drug that easily forms a hydrate.

A drug that does not easily form a hydrate in the context of the present specification refers to drugs other than the above-mentioned drug that easily forms a hydrate. Specific examples of the drug that does not easily form a hydrate include miconazole, isosorbide dinitrate, ibuprofen, diclofenac, edaravone, tulobuterol, donepezil and pharmaceutically acceptable salts thereof.

When a drug does not easily form a hydrate is used, crystal formation of the drug can be suppressed even without storage of a preparation under a dry atmosphere. Therefore, the present invention can be practiced more advantageously. That is, using a drug that does not easily form a hydrate, crystal formation of the drug dissolved in an adhesive layer during the heating step of a patch preparation precursor can be suppressed irrespective of storage of the patch preparation after production under high humidity conditions or low humidity conditions. On the other hand, when a drug that easily forms a hydrate is used, the drug in an adhesive layer may form a hydrate when the patch preparation after production is stored under high humidity conditions. In this case, even when crystals of the drug are dissolved in the heating step of a patch preparation precursor, a hydrate crystal of the drug may be formed.

The present invention is explained in more detail in the following by referring to Examples. The present invention is not limited by the following Examples, and various modifications may be made without departing from the spirit of the present invention before practicing the invention, which are all encompassed in the technical scope of the present invention. In the following description, unless otherwise specified, "parts" and "%" mean "parts by weight" and "wt%", respectively.

1. Preparation of polymer solution

(1) Preparation of acrylic polymer solution

Under an inert gas atmosphere, acrylic acid 2-ethylhexyl (75 parts), N-vinyl-2-pyrrolidone (22 parts), acrylic acid (3 parts) and azobisisobutyronitrile (0.2 parts) were added to ethyl acetate, and the mixture was subjected to solution polymerization at 60°C to give a polymer solution (polymer solid content: 28%).

(2) Preparation of polyisobutylene polymer solution

High molecular weight polyisobutylene (viscosity average molecular weight 4000000, Oppanol(R) B200, manufactured by BASF, 22.0 parts as solid content), low molecular weight polyisobutylene (viscosity average molecular weight 55000, Oppanol(R) B12, manufactured by BASF, 38.0 parts as solid content), and alicyclic saturated hydrocarbon resin (softening point 140°C, ARKON(R) P-140, manufactured by Arakawa Chemical Industries, Ltd., 40.0 parts) were dissolved in toluene to give a polyisobutylene polymer solution (polymer solid content: 21%).

2. Examples 1 - 19, Comparative Examples 1 - 7 and Reference Examples 1 - 2

Starting materials were mixed in the amounts shown in Tables 1 and 2 to give compositions for forming an adhesive layer, which were applied to one surface of a polyethylene terephthalate film (hereinafter to be indicated as PET, thickness 75 $\mu$m) as a release liner such that the thickness after drying was 200 $\mu$m, and dried to form an adhesive layer. To the adhesive layer was adhered a non-woven fabric surface of a PET film (thickness 2 $\mu$m)-PET non-woven fabric (fabric weight 12 g/m$^2$) laminate as a support, and the resulting product was subjected to an aging treatment (crosslinking treatment of the adhesive layer) at 70°C for 48 hr to give a laminated sheet. The laminated sheet after the aging treatment was cut into a shape of a patch preparation, and packed in a packing container under an atmosphere of oxygen concentration not more than 3% to give a patch preparation precursor. In Examples 7 - 19 and Comparative Examples 5 - 7, a PET film (thickness 25 $\mu$m) was used as a support, the thickness of the adhesive layer after drying was 100 $\mu$m, and the aging treatment (crosslinking treatment of the adhesive layer) was omitted. These changes do not influence the presence or absence of the crystal formation of the drug.

The obtained patch preparation precursors were heated at the temperature and time of the heating conditions shown in Tables 1 and 2, and cooled by itself to the environmental temperature to give the patch preparations of Examples 1 - 19, Comparative Examples 1 - 7 and Reference Examples 1 and 2. The melting point of each drug and whether or not a crystallization peak (indicated as Tc in the Tables) was detected by DSC are shown in Tables 1 and 2.

3. Reference Example 3

In the same manner as in Example 3 except that, in Example 3, the cutting step was omitted by forming an adhesive layer by casting a composition for forming an adhesive layer into a container with a shape of a patch preparation and after a release treatment of the inside, such that the thickness of the adhesive layer after drying was 200 μm, and drying same, and the heating step was omitted, the patch preparation of Reference Example 3 was obtained.
Abbreviations in Tables 1 and 2 mean the following.

acrylic: acrylic polymer
PIB: polyisobutylene polymer
SIS: styrene-isoprene-styrene rubber (Kraton D1161JP, Kraton Performance Polymers Inc. Japan)
IPM: isopropyl myristate (CRODAMOL IPM, CRODA Japan K.K.)
ODO: octyldodecanol (RISONOL SP, Kokyu Alcohol Kogyo Co., Ltd.)
COCONARD RK: caprylic acid triglyceride (COCONARD RK, Kao Corporation)

<Experimental Example>

4. Observation of crystal formation

The patch.preparation packed in a packing container immediately after production was stored in an incubator at 5°C or 25°C for 6 months. During the storage period for 6 months, the patch preparation was taken out from the incubator, and observed for crystal formation of the drug on the adhesive face of the adhesive layer and the inside thereof by visual observation and optical microscopic observation (magnification: 100-fold). A patch preparation that did not show crystal formation of the drug on the adhesive face of the adhesive layer and the inside thereof by visual and optical microscopic observation (magnification 100-fold) was evaluated as O, a patch preparation that did not show crystal formation of the drug on the adhesive face of the adhesive layer and the inside thereof by visual observation but showed slight crystal formation by optical microscopic observation (magnification: 100-fold) was evaluated as Δ, and a patch preparation that showed clear crystal formation by visual observation was evaluated as x.

5. Evaluation of drug stability

In the patch preparations of Examples 1 - 6, Comparative Examples 1 - 3, and Reference Examples 1 - 2, the drugs in the patch preparations immediately after production were extracted with methanol, and analyzed by high performance liquid chromatography (hereinafter to be referred to as HPLC) to evaluate the impurity rate (in HPLC chart, the ratio of summation of peak area corresponding to drug impurity to peak area corresponding to drug) of the drug. A patch preparation that showed an impurity rate of not less than 0.5% was evaluated as x, and a patch preparation that showed an impurity rate of less than 0.5% was evaluated as O.

6. Evaluation of drug release property

With regard to the patch preparations of Examples 1 - 6, Comparative Examples 1 - 3, and Reference Examples 1 - 2, the drug release property of the patch preparations packed in packing containers was evaluated after storage of the patch preparations at 25°C for 6 months. The release test of the patch preparations was performed according to U.S. Pharmacopeia 26, <724> Drug Release, Transdermal Delivery Systems-General Drug Release Standards, and a release solution was taken after 0.25, 0.5, 4 and 24 hr from the start of the test. The recovered'release solution was filtered with a membrane filter, quantified by HPLC, and measured for the amount of the released drug. The drug release rate was calculated as the percentage of the amount of the drug (weight) released after a given time relative to the amount of the drug (weight) contained in the patch preparation before the drug release.
The drug release rate after a given time of the patch preparation stored at 25°C for 6 months and that of the patch preparation immediately after production were compared, and a patch preparation without change was evaluated as O, and a patch preparation with change was evaluated as x. Here, "without change" means that the difference between a patch preparation stored at 25°C for 6 months and a patch preparation immediately after production was within 3% in all drug release rates that were taken after 0.25 - 4 hr from the start of the test.

7. Color change

With regard to the patch preparations of Examples 1 - 6, Comparative Examples 1 - 3, and Reference Examples 1 - 2, the color value of a patch preparation immediately after production was measured in the CIE1976(L*a*b*) color system with a color difference meter (manufactured by Konica Minolta Holdings, Inc., model number CR-400) and according to the attached manual.

Using the color value of Comparative Example 1 as a standard color, color change ΔE of Examples 1 - 6, Comparative Examples 2 - 3 and Reference Examples 1 - 2 was calculated using the formula (I) shown below.

In the formula, (L*1,a*1,b*1) is a color value of the standard color, and (L*2,a*2,b*2) is a color value of the evaluation subject. A patch preparation with a ΔE value of less than 2 was evaluated as O, and a patch preparation with a ΔE value of 2 or more was evaluated as x.

$$\triangle E = \{(L*2 - L*1)^2 + (a*2 - a*1)^2 + (b*2 - b*1)^2\}^{1/2} \quad \cdots (I)$$

8. Measurement of saturating concentration in adhesive layer

Patch preparations having an incrementing drug weight concentration by 1 wt% were prepared, and a liner on the adhesive layer of each patch preparation was detached. After standing the drug on the surface of the adhesive layer, the liner was laminated again on the adhesive layer and the resulting product was stored in an incubator at 25°C for 6 months. The patch preparation was taken out from the incubator every one month during the preservation for 6 months, and size change of the standing drug was visually and optical microscopically observed (magnification 500-fold). The maximum concentration with no change of the drug size was judged as the saturating concentration in the adhesive layer of the drug.

When the saturating concentration was evaluated as less than 1% (the size of the standing drug grows even in a patch preparation having a drug concentration of 1 wt%), patch preparations having an incrementing drug concentration by 0.2 wt% were prepared within the drug concentration range of 0 - 1 wt%, and a saturating concentration was measured again in the same manner as above.

9. Crystal formation of drug in adhesive layer due to physical stimulation

An adhesive layer of the patch preparation was pierced through with a cutter to give a physical stimulation. These were packed in packing containers and preserved at 25°C and 60%RH for 6 months. To determine whether a crystal of the drug was formed in the adhesive layer before physical stimulation, immediately after application of physical stimulation (not less than 0 hr and within 24 hr after physical stimulation), and after physical stimulation (not less than 24 hr and within 6 months after physical stimulation), the adhesive face of the adhesive layer and the inside thereof were observed visually and with an optical microscope. A patch preparation with crystal formation by visual observation and optical microscopic observation (magnification 500-fold) was evaluated as Y, a patch preparation without crystal formation by visual observation and optical microscopic observation (magnification 500-fold) was evaluated as N, and a patch preparation without crystal formation by visual observation but with crystal formation by optical microscopic observation (500-fold) was evaluated as S.

The above-mentioned results are shown in Table 3.

Table 1

| | polymer (solid content) | | liquid component | | crosslinking agent | additive | | drug | | | | | | condition of heating step |
|---|---|---|---|---|---|---|---|---|---|---|---|---|---|---|
| | kind | amount (parts) | kind | amount (parts) | amount (parts) | kind | amount (parts) | drug name | weight concentration (wt %) | Tc | melting point (°C) | saturating concentration (wt%) | The ratio of the weight concentration to the saturating concentration | (temperature, time) |
| Comp. Ex. 1 | acrylic | 40.9 | IPM | 49.8 | 0.2 | NaOH | 0.8 | donepezil hydrochloride | 8.3 | n.d. | 92 | 7 | 119% | heating none |
| Comp. Ex. 2 | acrylic | 40.9 | IPM | 49.8 | 0.2 | NaOH | 0.8 | donepezil hydrochloride | 8.3 | n.d. | 92 | 7 | 119% | 30°C 200h |
| Ex. 1 | acrylic | 40.9 | IPM | 49.8 | 0.2 | NaOH | 0.8 | donepezil hydrochloride | 8.3 | n.d. | 92 | 7 | 119% | 50°C 12h |
| Comp. Ex. 3 | acrylic | 40.9 | IPM | 49.8 | 0.2 | NaOH | 0.8 | donepezil hydrochloride | 8.3 | n.d. | 92 | 7 | 119% | 60°C 3h |
| Ex. 2 | acrylic | 40.9 | IPM | 49.8 | 0.2 | NaOH | 0.8 | donepezil hydrochloride | 8.3 | n.d. | 92 | 7 | 119% | 60°C 6h |
| Ex. 3 | acrylic | 40.9 | IPM | 49.8 | 0.2 | NaOH | 0.8 | donepezil hydrochloride | 8.3 | n.d. | 92 | 7 | 119% | 60°C 12h |
| Ex. 4 | acrylic | 40.9 | IPM | 49.8 | 0.2 | NaOH | 0.8 | donepezil hydrochloride | 8.3 | n.d. | 92 | 7 | 119% | 60°C 30h |
| Ex. 5 | acrylic | 40.9 | IPM | 49.8 | 0.2 | NaOH | 0.8 | donepezil hydrochloride | 8.3 | n.d. | 92 | 7 | 119% | 60°C 72h |

(continued)

| | polymer (solid content) | | liquid component | | crosslinking agent | additive | | drug | | | | | | condition of heating step |
|---|---|---|---|---|---|---|---|---|---|---|---|---|---|---|
| | kind | amount (parts) | kind | amount (parts) | amount (parts) | kind | amount (parts) | drug name | weight concentration (wt %) | Tc | melting point (°C) | saturating concentration (wt%) | The ratio of the weight concentration to the saturating concentration | (temperature, time) |
| Ref. Ex.1 | acrylic | 40.9 | IPM | 49.8 | 0.2 | NaOH | 0.8 | donepezil hydrochloride | 8.3 | n.d. | 92 | 7 | 119% | 60°C 200h |
| Ex. 6 | acrylic | 40.9 | IPM | 49.8 | 0.2 | NaOH | 0.8 | donepezil hydrochloride | 8.3 | n.d. | 92 | 7 | 119% | 80°C 48h |
| Ref. Ex. 2 | acrylic | 40.9 | IPM | 49.8 | 0.2 | NaOH | 0.8 | donepezil hydrochloride | 8.3 | n.d. | 92 | 7 | 119% | 100°C 3h |
| Comp. Ex. 4 | acrylic | 45.5 | IPM | 49.8 | 0.2 | NaOH | 0.4 | donepezil hydrochloride | 4.1 | n.d. | 92 | 7 | 59% | 60°C 12h |

EP 2 377 515 A1

Table 2

| | polymer (solid content) | | liquid component | | crosslinking agent | additive | | drug | | | | | | condition of heating step |
|---|---|---|---|---|---|---|---|---|---|---|---|---|---|---|
| | kind | amount (parts) | kind | amount (parts) | amount (parts) | kind | amount (parts) | drug name | Weight concentration (wt%) | Tc | melting point (°C) | saturating concentration (wt%) | The ratio of the weight concentration to the saturating concentration | (temperature, time) |
| Ex. 7 | PIB | 68 | IPM | 30 | - | - | - | donepezilbase | 2 | n.d. | 92 | 0.8 | 250% | 60°C 12h |
| Ex. 8 | PIB | 68 | liquid paraffin | 30 | - | - | - | donepezilbase | 2 | n.d. | 92 | 0.8 | 250% | 60°C 12h |
| Ex.9 | PIB | 67 | ODO | 30 | - | - | - | donepezilbase | 3 | n.d. | 92 | 2 | 150% | 60°C 12h |
| Ex.10 | SIS | 68 | liquid paraffin | 30 | - | - | - | donepezilbase | 2 | n.d. | 92 | 1 | 200% | 60°C 12h |
| Comp. Ex. 5 | acrylic | 47.4 | IPM | 47.4 | 0.2 | - | - | miconazole | 5 | n.d. | 83 | 9 | 56% | 60°C 12h |
| Ex.11 | acrylic | 40.4 | IPM | 40.4 | 0.2 | - | - | miconazole | 19 | n.d. | 83 | 9 | 211% | 60°C 12h |
| Comp. Ex. 6 | acrylic | 34.4 | IPM | 34.5 | 0.1 | - | - | miconazole | 31 | n.d. | 83 | 9 | 344% | 60°C 12h |
| Comp. Ex. 7 | acrylic | 29.4 | IPM | 29.5 | 0.1 | - | - | miconazole | 41 | n.d. | 83 | 9 | 456% | 60°C 12h |
| Ex.12 | acrylic | 42.4 | IPM | 42.4 | 0.2 | - | - | isosorbide dinitrate | 15 | n.d. | 70 | 8 | 188% | 60°C 12h |
| Ex. 13 | acrylic | 35.4 | IPM | 35.5 | 0.1 | - | - | ibuprofen | 29 | n.d. | 75 | 23 | 126% | 60°C 12h |
| Ex. 14 | acrylic | 44.4 | IPM | 44.4 | 0.2 | - | - | diclofenac | 11 | n.d. | 168 | 9 | 122% | 60°C 12h |
| Ex. 15 | acrylic | 38.4 | ODO | 38.4 | 0.2 | - | - | miconazole | 23 | n.d. | 83 | 19 | 121% | 60°C 12h |
| Ex.16 | acrylic | 38.4 | COCONARDRK | 38.4 | 0.2 | - | - | miconazole | 23 | n.d. | 83 | 19 | 121% | 60°C 12h |
| Ex. 17 | PIB | 67 | IPM | 30 | | - | - | miconazole | 3 | n.d. | 83 | 1 | 300% | 60°C 12h |
| Ex. 18 | acrylic | 46.9 | IPM | 46.9 | 0.2 | - | - | edaravone | 6 | detected | 127 | 3 | 200% | 60°C 12h |
| Ref. Ex. 3 | acrylic | 40.9 | IPM | 49.8 | 0.2 | NaOH | 0.8 | donepezil hydrochloride | 8.3 | n.d. | 92 | 7 | 119% | none |
| Ex. 19 | acrylic | 40.0 | IPM | 40.0 | 0.2 | - | - | scopolamine | 20.0 | n.d. | 68 | - | - | 60°C 12h |

EP 2 377 515 A1

Table 3

| | storage before physical stimulation | | | | | relationship between physical stimulation and crystal formation | | |
| | crystal observation | | release characteristic | drug stability | color change | before physical stimulation | immediately after physical stimulation (25˚C, 60%RH less than 1 day) | preservation after physical stimulation (25˚C, 60%RH 1 day - 6 months) |
| | 5˚C 6 month storage | 25˚C 6 month storage | | | | | | |
|---|---|---|---|---|---|---|---|---|
| Comp. Ex. 1 | × | × | × | ○ | (standard color) | Y | Y | Y |
| Comp.Ex.2 | × | × | × | ○ | ○ | Y | Y | Y |
| Ex.1 | Δ | ○ | ○ | O | O | N | N | Y |
| Comp.Ex.3 | × | × | x | O | O | Y | Y | Y |
| Ex. 2 | Δ | O | O | O | O | N | N | Y |
| Ex. 3 | O | O | O | O | O | N | N | Y |
| Ex. 4 | O | O | O | O | O | N | N | Y |
| Ex. 5 | O | O | O | O | O | N | N | Y |
| Ref. Ex. 1 | O | O | O | × | × | N | N | Y |
| Ex. 6 | O | O | O | O | O | N | N | Y |
| Ref. Ex. 2 | O | O | O | × | × | N | N | Y |
| Comp.Ex.4 | ○ | ○ | - | - | - | N | N | N |
| Ex. 7 | ○ | ○ | - | - | - | N | N | Y |
| Ex. 8 | ○ | ○ | - | - | - | N | N | Y |
| Ex. 9 | ○ | ○ | - | - | - | N | N | Y |
| Ex. 10 | ○ | ○ | - | - | - | N | N | Y |
| Comp. Ex. 5 | ○ | ○ | - | - | - | N | N | N |
| Ex.11 | ○ | ○ | - | - | - | N | N | Y |
| Comp.Ex.6 | ○ | ○ | - | - | - | N | Y | Y |
| Comp.Ex.7 | × | × | - | - | - | Y | Y | Y |

17

(continued)

| | storage before physical stimulation | | | | | relationship between physical stimulation and crystal formation | | |
|---|---|---|---|---|---|---|---|---|
| | crystal observation | | release characteristic | drug stability | color change | before physical stimulation | immediately after physical stimulation (25˚C, 60%RH less than 1 day) | preservation after physical stimulation (25˚C, 60%RH 1 day - 6 months) |
| | 5˚C 6 month storage | 25˚C 6 month storage | | | | | | |
| Ex.12 | ○ | ○ | - | - | - | N | N | Y |
| Ex. 13 | ○ | ○ | - | - | - | N | N | Y |
| Ex. 14 | ○ | ○ | - | - | - | N | N | Y |
| Ex.15 | ○ | ○ | - | - | - | N | N | Y |
| Ex.16 | ○ | ○ | - | - | - | N | N | Y |
| Ex.17 | ○ | ○ | - | - | - | N | N | Y |
| Ex. 18 | △ | △ | - | - | - | S | S | Y |
| Ref. Ex. 3 | ○ | ○ | - | - | - | N | N | Y |

○: no crystal by visual observation and no crystal with microscope (100-fold)

△ : no crystal by visual observation and presence of crystal with microscope (100-fold)

✕: presence of crystal by visual observation N: no crystal by visual observation and no crystal with microscope (500-fold) S: no crystal by visual observation and presence of crystal with microscope (500-fold) Y: presence of crystal by visual observation

From the test results, the following consideration was obtained.

By comparison of Comparative Example 3 and Example 3 produced via the patch preparation precursors produced in the same formulation, in Comparative Example 3 obtained by heating the patch preparation precursor at 60˚C for 3 hr, crystals were formed, while in Example 3 obtained by heating the patch preparation precursor at 60˚C for 12 hr, a crystal was not formed. In addition, Reference Example 1 produced by heating the same patch preparation precursor as those of Comparative Example 3 and Example 3 at 60˚C for 200 hr did not form a crystal. However, the drug stability was low, and color change was marked. Therefore, when the heating time of the patch preparation precursor is too short, the crystal core formed in the production step cannot be dissolved completely, and crystal formation during storage of the preparation after production cannot be completely prevented. Moreover, when the heating time of the patch preparation precursor is too long, the drug was degraded, and drug stability and appearance were impaired, though crystal was not formed during storage of the preparation after production.

In addition, in Comparative Example 2 obtained by heating the patch preparation precursor at 30˚C for 200 hr, crystals were formed during storage of the patch preparation after production. Furthermore, in Reference Example 2 obtained by heating the patch preparation precursor at 100˚C for 3 hr, a crystal was not formed during storage of the patch preparation after production. However, the drug was degraded, and drug stability and appearance were impaired. Therefore, it has been clarified that a low heating temperature of the patch preparation precursor results in a failure to completely dissolve crystals of the drug generated in the production step, and a high heating temperature of the patch preparation precursor leads to degradation of the drug in the patch preparation.

In Example 11, a crystal was not formed immediately after application of a physical stimulation to the adhesive layer. However, a crystal of the drug was formed during preservation after the physical stimulation. On the other hand, in Comparative Example 5, a crystal was not formed during preservation after application of physical stimulation. In Comparative Example 5, sufficient skin permeability of the drug could not be achieved, since the drug concentration in the patch was lower than that in Example 11.

In Comparative Example 6, a crystal of the drug was formed in the adhesive layer within 24 hr after application of a physical stimulation. Therefore, Comparative Example 6 permits easy crystal formation of the drug, and it is expected that crystals will cover the surface of the adhesive layer before actually applying the patch preparation to the skin of patients. As a result, the adhesion area will be decreased, and the adhesiveness to the skin will also be decreased. Furthermore, in Comparative Example 7, crystals were already formed even before application of a physical stimulation and the adhesiveness to the skin decreased as in Comparative Example 6, which prevents easy use of the patch preparation.

In Example 18, a crystal of the drug was slightly observed before application of a physical stimulation, and within 24 hr after the physical stimulation. However, the amount of crystal was sufficiently small and did not influence adhesiveness to the skin during use. That the crystal was slightly observed means absence of crystal by visual observation, but found under an optical microscope (500-fold). In addition, since crystals were observed 24 hr after and within 6 months of the physical stimulation by visual observation, sufficient skin permeability of the drug was afforded.

From the results of Example 11 - Example 19, it is appreciated that in a patch preparation in which a ratio of the weight concentration to the saturating concentration is 100 - 300%, crystals do not form immediately after application of a physical stimulation to the adhesive layer, but crystals are precipitated within 6 months, irrespective of the polymer, liquid component and drug contained in the patch preparation.

In Example 3, a crystal of the drug was formed in the adhesive layer due to application of a physical stimulation to the adhesive layer. However, in Comparative Example 4, a crystal was not formed. In Reference Example 3 without cutting processing of the adhesive layer, a crystal of the drug was not formed before physical stimulation. However, preservation after physical stimulation to the adhesive layer resulted in the crystal formation of the drug. In Example 3 and Reference Example 3, crystal formation of the drug in the adhesive layer was not observed at 24 hr after the start of the preservation. These results indicate that drug concentration and skin permeability of the drug are low in Comparative Example 4 that did not show crystal precipitation even with physical stimulation, and skin permeability of the drug is sufficient in Example 3 that showed crystal precipitation due to physical stimulation.

To investigate the influence of humidity during storage of the patch preparation after production on crystal formation, the patch preparation of Example 12 was subjected to a further storage test at high humidity and low humidity. The high humidity storage test was performed by storing a patch preparation immediately after production, in an incubator at 25˚C (high humidity (humidity: 75%RH)) for 1 month without packing in a packing container, crystal formation was observed and evaluated in the same manner as in the above-mentioned "4. Observation of crystal formation". The low humidity storage test was performed by storing a patch preparation immediately after production, in an incubator at 25˚C (low humidity (humidity: 10%RH)) for 1 month without packing in a packing container, crystal formation was observed and evaluated in the same manner as in the above-mentioned "4. Observation of crystal formation". Thereafter, the adhesive layer was cut through with a cutter to apply physical stimulation, and the adhesive layer was preserved under the same conditions as before the physical stimulation. The adhesive face of the adhesive layer and the inside thereof were observed with an optical microscope (magnification 500-fold) before physical stimulation, immediately after physical stimulation (not less than 0 hr and within 24 hr after physical stimulation), and after physical stimulation (not less than 24 hr and within 6 months after physical stimulation). A patch preparation with crystal formation was evaluated as Y, and a patch preparation without crystal formation was evaluated as N. The results are shown in Table 4. Isosorbide dinitrate is a drug that does not form a hydrate with ease.

[Table 4]

| | storage before physical stimulation | | relationship between physical stimulation and crystal formation | | |
| --- | --- | --- | --- | --- | --- |
| | crystal observation | | before physical stimulation | immediately after physical stimulation (25°C, 60%RH less than 1 day) | preservation after physical stimulation (25°C, 60%RH 1 day - 6 months) |
| | 25°C, 6 month storage high humidity conditions | 25°C, 6 month storage low humidity conditions | | | |
| Ex. 12 | ○ | - | N | N | Y |
| Ex. 12 | - | ○ | N | N | Y |
| Ex. 19 | - | ○ | N | N | Y |

From Table 4, it is clear that the patch preparation containing isosorbide dinitrate, in which a hydrate crystal was not formed with ease (Example 12), affords sufficient skin permeability even when the patch preparation after production is stored in high humidity.

## Claims

1. A patch preparation comprising: a support; and an adhesive layer containing a drug on one surface of the support, wherein a crystal of the drug is not formed in the adhesive layer immediately after application of a physical stimulation to the adhesive layer, while a crystal of the drug is formed during preservation after the physical stimulation.

2. The patch preparation of claim 1, wherein the drug in the adhesive layer is dissolved in the adhesive layer.

3. The patch preparation of claim 1 or 2, wherein a crystal of the drug is formed not less than 24 hr and within 6 months when the patch preparation is preserved at not more than 25°C after application of physical stimulation.

4. A method of producing the patch preparation of any one of claims 1 to 3, comprising a step of heating the adhesive layer containing the drug to a temperature less than the melting point of the drug.

Europäisches
Patentamt

European
Patent Office

Office européen
des brevets

# EUROPEAN SEARCH REPORT

Application Number

EP 11 16 0476

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 0 922 453 A2 (NITTO DENKO CORP [JP]; HOKURIKU PHARMACEUTICAL [JP] NITTO DENKO CORP []) 16 June 1999 (1999-06-16) * paragraphs [0005] - [0011], [0052], [0054] * | 1-4 | INV. A61K9/00 A61K9/70 |
| X | WO 2009/145177 A1 (NITTO DENKO CORP [JP]; SEKIYA JUNICHI [JP]; HANATANI AKINORI [JP]; TER) 3 December 2009 (2009-12-03) * claims 1-4 * | 1-4 | |
| X | EP 0 677 290 A1 (NITTO DENKO CORP [JP]; HOKURIKU PHARMACEUTICAL [JP]) 18 October 1995 (1995-10-18) * claim 1 * | 1-4 | |
| X | EP 2 016 941 A1 (HISAMITSU PHARMACEUTICAL CO [JP]) 21 January 2009 (2009-01-21) * claims 1-5 * | 1-4 | |
| X | EP 0 661 046 A2 (ROTTA RESEARCH LAB [IT] ROTTA RES BV [NL]) 5 July 1995 (1995-07-05) * example 5 * | 1-4 | TECHNICAL FIELDS SEARCHED (IPC) A61K |
| A | WO 2006/082728 A1 (HISAMITSU PHARMACEUTICAL CO [JP]; AIDA KAZUNOSUKE [JP]; MICHINAKA YASU) 10 August 2006 (2006-08-10) * examples 2-3 * | 1-4 | |
| A | US 7 056 528 B1 (BRACHT STEFAN [DE]) 6 June 2006 (2006-06-06) * claim 1 * | 1-4 | |

The present search report has been drawn up for all claims

| Place of search | Date of completion of the search | Examiner |
|---|---|---|
| Munich | 21 June 2011 | Giese, Hans-Hermann |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 11 16 0476

This annex lists the patent family members relating to the patent documents cited in the above-mentioned European search report.
The members are as contained in the European Patent Office EDP file on
The European Patent Office is in no way liable for these particulars which are merely given for the purpose of information.

21-06-2011

| Patent document cited in search report | | Publication date | Patent family member(s) | | Publication date |
|---|---|---|---|---|---|
| EP 0922453 | A2 | 16-06-1999 | AT | 305775 T | 15-10-2005 |
| | | | BR | 9806470 A | 11-04-2000 |
| | | | CA | 2255447 A1 | 12-06-1999 |
| | | | CN | 1226422 A | 25-08-1999 |
| | | | DE | 69831785 T2 | 13-07-2006 |
| | | | DK | 0922453 T3 | 07-11-2005 |
| | | | ES | 2246062 T3 | 01-02-2006 |
| | | | HK | 1022091 A1 | 27-08-2004 |
| | | | HU | 9802889 A2 | 28-06-1999 |
| | | | JP | 3930984 B2 | 13-06-2007 |
| | | | JP | 11228395 A | 24-08-1999 |
| | | | PL | 330212 A1 | 21-06-1999 |
| | | | PT | 922453 E | 30-11-2005 |
| | | | US | 6117447 A | 12-09-2000 |
| WO 2009145177 | A1 | 03-12-2009 | CA | 2724502 A1 | 03-12-2009 |
| | | | CN | 102014905 A | 13-04-2011 |
| | | | EP | 2279739 A1 | 02-02-2011 |
| | | | KR | 20110011650 A | 08-02-2011 |
| | | | US | 2011056863 A1 | 10-03-2011 |
| EP 0677290 | A1 | 18-10-1995 | AT | 203158 T | 15-08-2001 |
| | | | CA | 2146723 A1 | 15-10-1995 |
| | | | DE | 69521734 D1 | 23-08-2001 |
| | | | DE | 69521734 T2 | 08-11-2001 |
| | | | ES | 2160647 T3 | 16-11-2001 |
| | | | JP | 2753800 B2 | 20-05-1998 |
| | | | JP | 7285854 A | 31-10-1995 |
| | | | US | 5639472 A | 17-06-1997 |
| EP 2016941 | A1 | 21-01-2009 | JP | 2007302582 A | 22-11-2007 |
| | | | WO | 2007129712 A1 | 15-11-2007 |
| | | | US | 2009175929 A1 | 09-07-2009 |
| EP 0661046 | A2 | 05-07-1995 | DE | 69413661 D1 | 05-11-1998 |
| | | | DE | 69413661 T2 | 17-06-1999 |
| | | | ES | 2122140 T3 | 16-12-1998 |
| WO 2006082728 | A1 | 10-08-2006 | US | 2008138388 A1 | 12-06-2008 |
| US 7056528 | B1 | 06-06-2006 | AT | 299019 T | 15-07-2005 |
| | | | AU | 7662400 A | 30-04-2001 |
| | | | CA | 2387143 A1 | 26-04-2001 |
| | | | DE | 19950066 A1 | 26-04-2001 |
| | | | WO | 0128531 A1 | 26-04-2001 |
| | | | EP | 1220662 A1 | 10-07-2002 |

EPO FORM P0459

For more details about this annex : see Official Journal of the European Patent Office, No. 12/82

**ANNEX TO THE EUROPEAN SEARCH REPORT
ON EUROPEAN PATENT APPLICATION NO.** EP 11 16 0476

This annex lists the patent family members relating to the patent documents cited in the above-mentioned European search report.
The members are as contained in the European Patent Office EDP file on
The European Patent Office is in no way liable for these particulars which are merely given for the purpose of information.

21-06-2011

| Patent document cited in search report | Publication date | Patent family member(s) | | Publication date |
|---|---|---|---|---|
| US 7056528 B1 | | ES | 2245648 T3 | 16-01-2006 |
| | | JP | 2003528037 T | 24-09-2003 |
| | | PT | 1220662 E | 31-10-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3566301 B
- JP 2006513195 A
- JP 2610314 B
- JP 4166276 B
- JP 2001514213 A